Europäisches Patentamt
European Patent Office
Office européen des brevets

(19) (11) Veröffentlichungsnummer: **0 347 679 A1**

(12) **EUROPÄISCHE PATENTANMELDUNG**

(21) Anmeldenummer: 89110529.8

(22) Anmeldetag: 10.06.89

(51) Int. Cl.⁴: C07C 69/712 , C07C 43/275 , C07D 213/64 , A01N 39/02 , A01N 43/40

(30) Priorität: 24.06.88 DE 3821389

(43) Veröffentlichungstag der Anmeldung: 27.12.89 Patentblatt 89/52

(84) Benannte Vertragsstaaten: BE CH DE FR GB IT LI NL

(71) Anmelder: BAYER AG

D-5090 Leverkusen 1 Bayerwerk(DE)

(72) Erfinder: Haug, Michael, Dr.
Fahner Weg 5
D-5060 Bergisch-Gladbach 2(DE)
Erfinder: Andree, Roland, Dr.
Schillerstrasse 17
D-4018 Langenfeld(DE)
Erfinder: Santel, Hans-Joachim, Dr.
Grünstrasse 9a
D-5090 Leverkusen 1(DE)
Erfinder: Schmidt, Robert R., Dr.
Im Waldwinkel 110
D-5060 Bergisch-Gladbach 2(DE)
Erfinder: Strang, Harry, Dr.
Unterdorfstrasse 6A
D-4000 Düsseldorf 31(DE)

(54) **1,7-Dioxy-naphthalin-Derivate.**

(57) Die Erfindung betrifft neue 1,7-Dioxy-naphthalin-Derivate, Verfahren und neue Zwischenprodukte zu ihrer Herstellung sowie ihre Verwendung als Herbizide.

Die neuen 1,7-Dioxy-naphthalin-Derivate entsprechen der allgemeinen Formel (I)

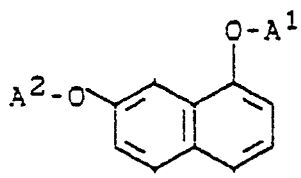

(I)

in welcher

$A^1$ und $A^2$ in jedem Einzelfall verschieden sind und jeweils der eine der beiden Reste ($A^1$ oder $A^2$) für die Gruppierung

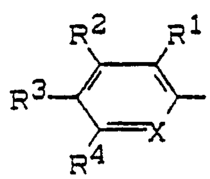

steht, und jeweils der andere der beiden Reste ($A^2$ oder $A^1$) für die Gruppierung $-A^3-Z$ steht, worin

$A^3$ für gegebenenfalls verzweigtes Alkandiyl steht und

Z für Cyano oder die Gruppierung -CO-Y steht,

(wobei die Reste $R^1$, $R^2$, $R^3$, $R^4$, X, Y und Z im einzelnen die in der Beschreibung angegebenen

EP 0 347 679 A1

Bedeutungen haben).

# 1,7-Dioxy-naphthalin-Derivate

Die Erfindung betrifft neue 1,7-Dioxy-naphthalin-Derivate, Verfahren und neue Zwischenprodukte zu ihrer Herstellung sowie ihre Verwendung als Herbizide.

Es ist bereits bekannt, daß bestimmte Dioxy-naphthalin-Derivate, wie z. B. α-(5-(3-Chlor-5-trifluormethyl-pyridin-2-yl-oxy)-naphthalin-1-yl-oxy)-propionsäure-ethylester herbizid wirksam sind (vgl. JP-A 79-32477/ Chem. Abstracts 91 (1979), 91510j). Die Wirkung dieser bekannten Verbindung gegen Unkräuter ist jedoch nicht immer ganz zufriedenstellend.

Es wurden nun neue 1,7-Dioxy-naphthalin-Derivate der allgemeinen Formel (I)

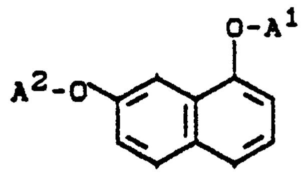

(I)

gefunden, in welcher
$A^1$ und $A^2$ in jedem Einzelfall verschieden sind und jeweils der eine der beiden Reste ($A^1$ oder $A^2$) für die Gruppierung

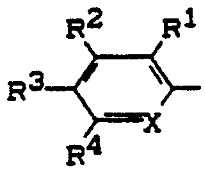

steht, worin
$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy, Trifluormethylthio oder Trifluormethylsulfonyl steht,
$R^4$ für Wasserstoff oder Halogen steht, und
X für Stickstoff oder die Gruppierung C-$R^5$ steht, worin
$R^5$ für Wasserstoff oder Halogen steht,
und jeweils der andere der beiden Reste ($A^2$ oder $A^1$) für die Gruppierung -$A^3$-Z steht, worin
$A^3$ für gegebenenfalls verzweigtes Alkandiyl steht und
Z für Cyano oder die Gruppierung -CO-Y steht, worin
Y für Halogen, Hydroxy, Amino, Alkylamino, Alkenylamino, Alkinylamino, Arylamino, Aralkylamino, Alkoxycarbonylalkylamino, Cyanamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung -O-$R^6$ steht, worin
$R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Arylthioalkyl, Arylalkoxyalkyl, Arylalkylthioalkyl, Trialkylsilylalkyl, Alkoxycarbonylalkyl, Alkylaminocarbonylalkyl, Arylaminocarbonylalkyl, N-Alkyl-N-aryl-aminocarbonylalkyl, Aralkyl, Azolylalkyl, Alkylidenamino oder für ein Ammonium-, Alkylammonium-, Alkalimetall-oder Erdalkalimetall-äquivalent steht oder für die Gruppierung

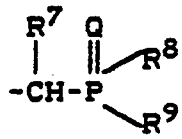

steht, worin
$R^7$ für Wasserstoff, Alkyl, Aryl, Furyl, Thienyl oder Pyridyl steht,
$R^8$ für Alkyl oder Alkoxy steht,

$R^9$ für Alkoxy steht und
Q für Sauerstoff oder Schwefel steht,
oder
$R^6$ weiterhin für die Gruppierung $-(CH_2)_n-R^{10}$ steht, worin
$R^{10}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und
n für die Zahlen 0, 1 oder 2 steht.

Die Erfindung betrifft sowohl die einzelnen möglichen isomeren Formen von Verbindungen der allgemeinen Formel (I) als auch die möglichen Gemische solcher isomeren Formen, insbesondere die R-Isomere.

Man erhält die neuen 1,7-Dioxy-naphthalin-Derivate der allgemeinen Formel (I), wenn man

(a) 7-substituierte 1-Naphthole der allgemeinen Formel (II)

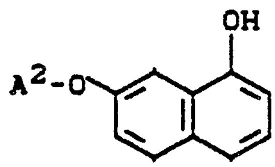

(II)

in welcher
$A^2$ die oben angegebene Bedeutung hat,
mit Verbindungen der allgemeinen Formel (III)
$Z^1 - A^1$ (III)
in welcher
$A^1$ die oben angegebene Bedeutung hat und
$Z^1$ für eine nucleophile Abgangsgruppe steht,
gegebenenfalls in Gegenwart eines Säureakzeptors und gegebenenfalls in Gegenwart eines Verdünnungsmittels umsetzt, und/oder

(b) 8-substituierte 2-Naphthole der allgemeinen Formel (IV)

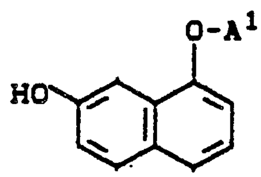

(IV)

in welcher
$A^1$ die oben angegebene Bedeutung hat,
mit Verbindungen der allgemeinen Formel (V)
$A^2 - Z^2$ (V)
in welcher
$A^2$ die oben angegebene Bedeutung hat und
$Z^2$ für eine nucleophile Abgangsgruppe steht
gegebenenfalls in Gegenwart eines Säureakzeptors und gegebenenfalls in Gegenwart eines Verdünnungsmittels umsetzt. und gegebenenfalls an den so erhaltenen Verbindungen der Formel (I) weitere Derivatisierungen in dem durch die obige Substituentendefinition vorgegebenen Rahmen nach üblichen Methoden durchführt.

Die neuen 1,7-Dioxy-naphthalin-Derivate der allgemeinen Formel (I) zeichnen sich durch starke herbizide Wirksamkeit aus.

Überraschenderweise zeigen die erfindungsgemäßen 1,7-Dioxy-naphthalin-Derivate der Formel (I) bei guter Verträglichkeit in wichtigen Kulturpflanzen gegen Problemunkräuter wesentlich stärkere Wirkung als $\alpha$-(5-(3-Chlor-5-trifluormethyl-pyridin-2-yl-oxy)-naphthalin-1-yl-oxy)propionsäureethylester, welches ein strukturell ähnlicher vorbekannter Wirkstoff gleicher Wirkungsrichtung ist.

Die Erfindung betrifft vorzugsweise Verbindungen der Formel (I), in welcher

$A^1$ und $A^2$ in jedem Einzelfall verschieden sind und jeweils einer der beiden Reste ($A^1$ oder $A^2$) für die Gruppierung

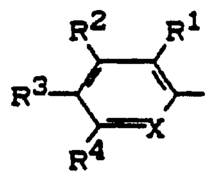

steht, worin
$R^1$ für Wasserstoff, Fluor, Chlor, Brom, Cyano oder Trifluormethyl steht,
$R^2$ für Wasserstoff, Fluor oder Chlor steht,
$R^3$ für Fluor, Chlor, Brom, Trifluormethyl, Trifluormethoxy, Trifluormethylthio oder Trifluormethylsulfonyl steht,
$R^4$ für Wasserstoff, Fluor oder Chlor steht,
X für Stickstoff oder die Gruppierung C-$R^5$ steht, worin
$R^5$ für Wasserstoff, Fluor, Chlor oder Brom steht,
und jeweils der andere der beiden Reste ($A^2$ oder $A^1$) für die Gruppierung -$A^3$-Z steht, worin
$A^3$ für gegebenenfalls verzweigtes $C_1$-$C_4$-Alkandiyl steht und
Z für Cyano oder die Gruppierung -CO-Y steht, worin
Y für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung -O-$R^6$ steht, worin
$R^6$ für einen gegebenenfalls durch Fluor und/ oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylaminocarbonyl-$C_1$-$C_2$-alkyl, Phenylaminocarbonyl-$C_1$-$C_4$-alkyl, N-($C_1$-$C_4$-Alkyl)-N-phenyl-aminocarbonyl-$C_1$-$C_4$-alkyl, Benzyl, Pyrazolyl-$C_1$-$C_4$-alkyl, $C_2$-$C_4$-Alkylidenamino oder für ein Ammonium-, ein $C_1$-$C_4$-Alkylammonium-, ein Natrium-, Kalium-oder Calcium-äquivalent steht, oder für die Gruppierung

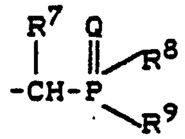

steht, worin
$R^7$ für Wasserstoff, $C_1$-$C_4$-Alkyl, Phenyl, Furyl, Thienyl oder Pyridyl steht,
$R^8$ für $C_1$-$C_4$-Alkyl oder $C_1$-$C_4$-Alkoxy steht,
$R^9$ für $C_1$-$C_4$-Alkoxy steht und
Q für Sauerstoff oder Schwefel steht,
oder
$R^6$ weiterhin für die Gruppierung $-(CH_2)_n-R^{10}$ steht, worin
n für die Zahlen 0, 1 oder 2 steht und
$R^{10}$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht.

Die Erfindung betrifft insbesondere Verbindungen der Formel (I), in welcher
$A^1$ und $A^2$ in jedem Einzelfall verschieden sind und jeweils einer der beiden Reste ($A^1$ oder $A^2$) für die Gruppierung

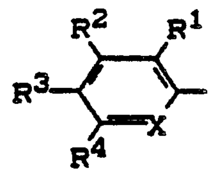

steht, worin
$R^1$ für Cyano, Fluor oder Chlor steht,
$R^2$ für Wasserstoff, Fluor oder Chlor steht,
$R^3$ für Chlor, Trifluormethyl oder Trifluormethylsulfonyl steht,
$R^4$ für Wasserstoff, Fluor oder Chlor steht,
X für Stickstoff oder die Gruppierung C-$R^5$ steht, worin
$R^5$ für Wasserstoff, Fluor oder Chlor steht,
und jeweils der andere der beiden Reste ($A^2$ oder $A^1$) für die Gruppierung -$A^3$-Z steht, worin
$A^3$ für Methylen ($-CH_2-$), Dimethylen ($-CH_2CH_2-$), Trimethylen ($(-CH_2-)_3$) oder Ethyliden

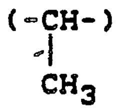

steht, und
Z für Cyano oder die Gruppierung -CO-Y steht, worin
Y für Chlor, Hydroxy, Amino, $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, Diallylamino, $C_1$-$C_4$-Alkylsulfonylamino, Phenylsulfonylamino, Hydroxyamino, Cyanamino, $C_1$-$C_4$-Alkoxyamino, Hydrazino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung -O-$R^6$ steht, worin
$R^6$ für $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-alkyl, $C_1$-$C_4$-Alkoxy-carbonyl-$C_1$-$C_2$-alkyl, $C_1$-$C_4$-Alkylamino-carbonyl-$C_1$-$C_2$-alkyl, Benzyl, Trimethylsilylmethyl oder für ein Ammonium-, $C_1$-$C_3$-Alkylammonium-, Natrium-, oder Kalium-äquivalent steht, oder für die Gruppierung

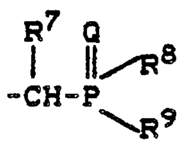

steht, worin
$R^7$ für Wasserstoff, Methyl, Phenyl, Furyl, Thienyl oder Pyridyl steht,
$R^8$ für Methoxy oder Ethoxy steht,
$R^9$ für Methoxy oder Ethoxy steht und
Q für Sauerstoff oder Schwefel steht
oder
$R^6$ weiterhin für die Gruppierung $(-CH_2-)_nR^{10}$ steht, worin
n für die Zahlen 0, 1 oder 2 steht und
$R^{10}$ für einen gegebenenfalls durch Chlor und/oder Methyl substituierten heterocyclischen Rest aus der Reihe Furyl, Tetrahydrofuryl, Thienyl, Perhydropyranyl, Oxazolyl, Thiazolyl und Dioxolanyl steht.

Verwendet man für das erfindungsgemäße Verfahren (a) beispielsweise 7-(3,5-Dichlor-pyridin-2-yl-oxy)-1-naphthol und α-Brom-propionsäure-ethylester als Ausgangsstoffe, so kann der Reaktionsablauf durch das folgende Formelschema wiedergegeben werden :

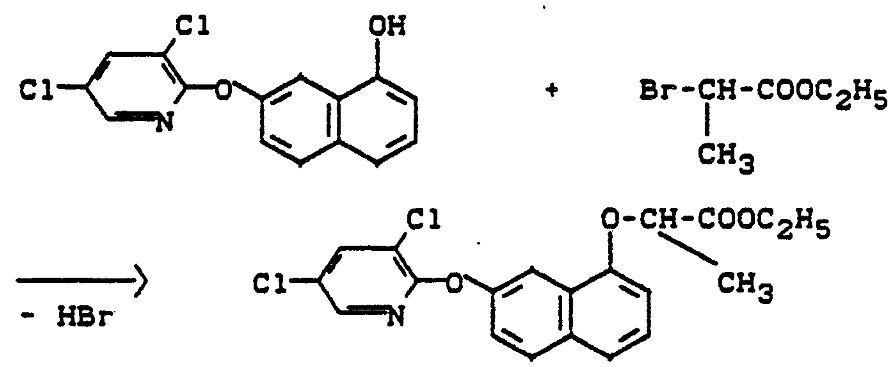


Verwendet man für das erfindungsgemäße Verfahren (b) beispielsweise 3,4,5-Trichlor-benzotrifluorid und (7-Hydroxy-naphthalin-1-yl-oxy)-essigsäure-propylester als Ausgangsstoffe, so kann der Reaktionsablauf durch das folgende Formelschema wiedergegeben werden:

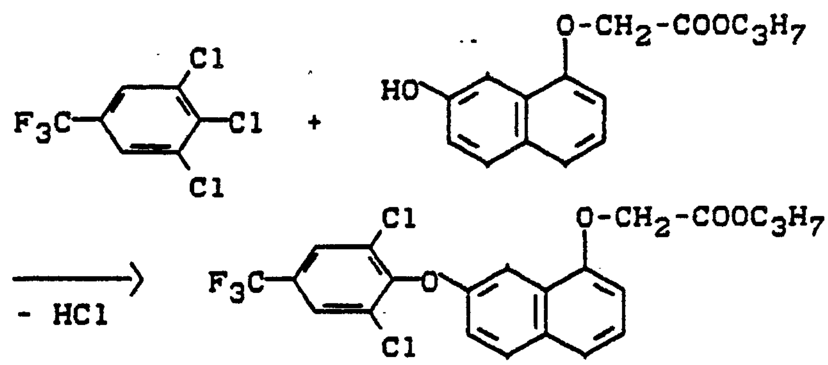


Die beim erfindungsgemäßen Verfahren (a) zur Herstellung von Verbindungen der Formel (I) als Ausgangsstoffe zu verwendenden 7-substituierten 1-Naphthole sind durch die Formel (II) allgemein definiert.

In Formel (II) hat $A^2$ vorzugsweise bzw. insbesondere diejenige Bedeutung, die bereits oben im Zusammenhang mit der Beschreibung der erfindungsgemäßen Verbindungen der Formel (I) vorzugsweise bzw. als insbesondere bevorzugt für $A^2$ angegeben wurde.

Als Beispiele für die Ausgangsstoffe der Formel (II) seien genannt:
7-(4-Trifluormethyl-phenoxy)-1-naphthol, 7-(2-Chlor-4-trifluormethyl-phenoxy)-1-naphthol, 7-(2,6-Dichlor-4-trifluormethyl-phenoxy)-1-naphthol, 7-(2-Chlor-6-fluor-4-trifluormethyl-phenoxy)-1-naphthol, 7-(2,6-Dichlor-3-fluor-4-trifluormethyl-phenoxy)-1-naphthol, 7-(2,3,6-Trichlor-4-trifluormethyl-phenoxy)-1-naphthol, 7-(3,5-Dichlorpyridin-2-yl-oxy)-1-naphthol; ferner α-(8-Hydroxy-naphthalin-2-yl-oxy)-propionsäure-nitril, -methylester, -ethylester, -propylester, -isopropylester, -butylester, -isobutylester und -sec-butylester, ß-(8-Hydroxy-naphthalin-2-yl-oxy)-propionsäure-nitril, -methylester, -ethylester, -propylester, -isopropylester, -butylester, -isobutylester und -sec-butylester, (8-Hydroxy-naphthalin-2-yl-oxy)-essigsäure-nitril, -methylester, -ethylester, -propylester, -isopropylester, -butylester, -isobutylester und -sec-butylester.

Die Ausgangsstoffe der Formel (II) sind noch nicht aus der Literatur bekannt. Man erhält die Verbindungen der Formel (II), wenn man Verbindungen der Formel (V)

$A^2$ - $Z^2$ (V)

in welcher

$A^2$ und $Z^2$ die oben angegebenen Bedeutungen haben,

mit 1,7-Dihydroxynaphthalin in Gegenwart eines Säureakzeptors, wie z. B. Natriumhydroxid oder Kaliumhydroxid, und in Gegenwart eines Verdünnungsmittels, wie z. B. Dimethylformamid, Dimethylacetamid, Dimethylsulfoxid, Tetramethylensulfon oder N-Methyl-pyrrolidon, bei Temperaturen zwischen -20 °C und 150 °C umsetzt und nach üblichen Methoden aufarbeitet.

Die beim erfindungsgemäßen Verfahren (b) zur Herstellung von Verbindungen der Formel (I) als Ausgangsstoffe zu verwendenden 8-substituierten 2-Naphthole sind durch die Formel (IV) allgemein definiert.

In Formel (IV) hat $A^1$ vorzugsweise bzw. insbesondere diejenige Bedeutung, die bereits oben im Zusammenhang mit der Beschreibung der erfindungsgemäßen Verbindungen der Formel (I) vorzugsweise

bzw. als insbesondere bevorzugt für $A^1$ angegeben wurde.

Als Beispiele für die Ausgangsstoffe der Formel (IV) seien genannt:

8-(4-Trifluormethyl-phenoxy)-2-naphthol, 8-(2-Chlor-4-trifluormethyl-phenoxy)-2-naphthol, 8-(2,6-Dichlor-4-trifluormethyl-phenoxy)-2-naphthol, 8-(2-Chlor-6-fluor-4-trifluormethyl-phenoxy)-2-naphthol, 8-(2,6-Dichlor-3-fluor-4-trifluormethyl-phenoxy)-2-naphthol, 8-(2,3,6-Trichlor-4-trifluormethyl-phenoxy)-2-naphthol, 8-(3,5-Dichlorpyridin-2-yl-oxy)-2-naphthol; ferner α-(7-Hydroxy-naphthalin-1-yl-oxy)-propionsäure-nitril, -methylester, -ethylester, -propylester, -isopropylester, -butylester, -isobutylester und -sec-butylester, ß-(7-Hydroxy-naphthalin-1-yl-oxy)-propionsäure-nitril, -methylester, -ethylester, -propylester, -isopropylester, -butylester, -isobutylester und -sec-butylester, (7-Hydroxy-naphthalin-1-yl-oxy)-essigsäure-nitril, -methylester, -ethylester, -propylester, -isopropylester, -butylester, -isobutylester und -sec-butylester.

Die Ausgangsstoffe der Formel (IV) sind noch nicht aus der Literatur bekannt. Man erhält die Verbindungen der Formel (IV), wenn man Verbindungen der Formel (III)

$Z^1$ - $A^1$ (III)

in welcher

$A^1$ und $Z^1$ die oben angegebenen Bedeutungen haben,

mit 1,7-Dihydroxynaphthalin in Gegenwart eines Säureakzeptors, wie z. B. Natriumhydroxid oder Kaliumhydroxid, und in Gegenwart eines Verdünnungsmittels, wie z. B. Dimethylformamid, Dimethylacetamid, Dimethylsulfoxid, Tetramethylensulfon oder N-Methyl-pyrrolidon, bei Temperaturen zwischen 20 °C und 150 °C umsetzt und nach üblichen Methoden aufarbeitet.

Die bei den erfindungsgemäßen Verfahren (a) und (b) weiter als Ausgangsstoffe zu verwendenden Verbindungen sind durch die Formeln (III) und (V) allgemein definiert.

In den Formeln (III) und (V) haben $A^1$ und $A^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $A^1$ und $A^2$ angegeben wurden und $Z^1$ und $Z^2$ stehen vorzugsweise für Fluor, Chlor, Brom, Iod, gegebenenfalls durch Fluor oder Chlor substituiertes $C_1$-$C_4$-Alkylsulfonyloxy oder gegebenenfalls durch Fluor, Chlor, Brom oder Methyl substituiertes Phenylsulfonyloxy, insbesondere für Chlor, Brom, Methylsulfonyloxy, Phenylsulfonyloxy oder 4-Methyl-phenylsulfonyloxy.

Als Beispiele für die Ausgangsstoffe der Formeln (III) und (IV) seien genannt:

4-Chlor-benzotrifluorid, 3,4-Dichlor-benzotrifluorid, 3,4,5-Trichlor-benzotrifluorid, 3,4-Dichlor-5-fluorbenzotrifluorid, 2,3,4,5-Tetrachlor-benzotrifluorid, 3,5-Dichlor-2,4-difluor-benzotrifluorid, 3-Chlor-4,5-difluor-benzotrifluorid und 2,3,5-Trichlor-pyridin; ferner Chloracetonitril, Bromacetonitril, ß-Brom-propionitril, ß-Chlor-propionitril, α-Chlor-, α-Brom-und α-Iod-propionsäure-methylester, -ethylester, -propylester, -isopropylester, -butylester, -isobutylester und -sec-butylester, ß-Chlor, ß-Brom- und ß-Iod-propionsäure-methylester, -ethylester, -propylester, -isopropylester, -butylester, -isobutylester und -sec-butylester, Chlor-, Brom- und Iod-essigsäure-methylester, -ethylester, -propylester, -isopropylester, -butylester, -isobutylester und -sec-butylester, α-Methylsulfonyloxy-, α-Ethylsulfonyloxy-, α-Propylsulfonyloxy-, α-Butylsulfonyloxy-, α-Trifluormethylsulfonyloxy-, α-Phenylsulfonyloxy- und α-(4-Methyl-phenylsulfonyloxy)-propionsäure-methylester, -ethylester, -propylester, -butylester, -isopropylester, -isobutylester und -sec-butylester.

Die Ausgangsstoffe der Formeln (III) und (V) sind bekannt und/oder können nach an sich bekannten Verfahren hergestellt werden (vgl. J. Chem. Soc. 1969, 211 - 217; ibid. 1971, 1547 - 1549; EP-A 34 402; US-P 4 424 396; EP-A 145 314; FR-A 2 538 380 (Chem. Abstracts 102 (1985), 61914x); DE-OS 27 58 002; DE-OS 28 54 542)).

Die erfindungsgemäßen Verfahren (a) und (b) zur Herstellung der neuen 1,7-Dioxy-naphthalin-Derivate der Formel (I) werden vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Als Säureakzeptoren können bei den erfindungsgemäßen Verfahren (a) und (b) alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Tri ethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahren (a) und (b) in einem größeren Bereich variiert werden. Im allgemeinen arbeitet man bei Temperaturen zwischen 0 °C und 200 °C, vorzugsweise bei Temperaturen zwischen 20 °C und 150 °C.

Die erfindungsgemäßen Verfahren (a) und (b) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a) und (b) werden die jeweils benötigten Ausgangsstoffe im allgemeinen in angenähert äquimolaren Mengen eingesetzt. Es ist jedoch auch möglich, eine der beiden jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren jeweils nach üblichen Methoden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u>

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u>

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u>

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u>

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfen-anlagen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen Kulturen, vor allem im Nachauflauf-Verfahren.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürlich und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoff, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage; ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 2-Chlor-4-ethylamino-6-isopropylamino-1,3,5-triazin (ATRAZIN); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N{[(4-methoxy-6-methyl-1,3,5-triazin-2-yl)-amino]-carbonyl}-benzosulfonamid (CHLORSULFURON); N,N-Dimethyl-N′-(3-chlor-4-methylphenyl)-harnstoff (CHLORTOLURON); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl-oder deren Ethylester (DICLOFOP); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6- fluor-2-pyridinyl)-oxy]-essigsäure bzw. deren 1-Methyl-heptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxy-benzonitril (IOXYNIL); N,N-Dimethyl-N′-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yl-oxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METSULFURON); N-(1-Ethylpropyl)-3,4-dimethyl-2,6-dinitroanilin (PENDIMETHALIN); 0-(6-Chlor-3-phenyl-pyridazin-4-yl)-S-octyl-thio-carbonat (PYRIDATE); 4-Ethylamino-2-t-butylamino-6-methylthio-s-triazin (TERBUTRYNE) und 3-[[[[(4-Methoxy-6-methyl-1,3,5-triazin-2-yl)-amino]-carbonyl]-amino]-sulfonyl]-thiophen-2-carbonsäure-methylester (THIAMETURON); Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln

ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granu late angewandet werden. Die Anwendung geschieht, in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Die Herstellung und die Verwendung der erfindungsgemäßen Wirkstoffe geht aus den nachfolgenden Beispielen hervor.

## Herstellungsbeispiele

### Beispiel 1

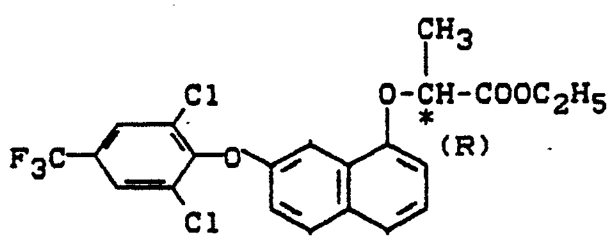

Eine Mischung aus 34,1 g (0,09 Mol) 7-(2,6-Dichlor-4-trifluormethyl-phenoxy)-1-naphthol, 17,1 g (0,063 Mol) (S)-α-(4-Methyl-phenylsulfonyloxy)-propionsäure-ethylester, 12,4 g Kaliumcarbonat und 150 ml Acetonitril wird 20 Stunden unter Rückfluß zum Sieden erhitzt. Nach dem Abkühlen auf 20 °C wird das Reaktionsgemisch mit 2N-Salzsäure verrührt und mit Toluol ausgeschüttelt. Die organische Phase wird abgetrennt, mit 2N-Natronlauge gewaschen, mit Natriumsulfat getrocknet und filtriert. Vom Filtrat wird das Lösungsmittel im Wasserstrahlvakuum sorgfältig abdestilliert.

Men erhält 12,8 g (30 % der Theorie) (R)-α-(7-(2,6-Dichlor-4-trifluormethyl-phenoxy)-naphthalin-1-yl-oxy)-propionsäureethylester als öligen Rückstand. $^1$H-NMR: δ = 7,6 ppm für

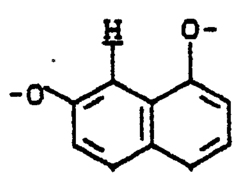

Analog Beispiel 1 und entsprechend der allgemeinen Beschreibung der erfindungsgemäßen Herstellungsverfahren können die in der nachstehenden Tabelle 1 aufgeführten Verbindungen der Formel (I) hergestellt werden:

**Tabelle 1:** Beispiele für die Verbindungen der Formel (I)

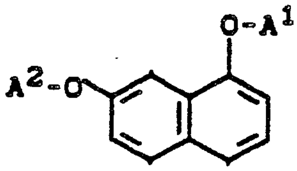

(I)

| Beisp.-Nr. | $A^2$ | $A^1$ | Physikalische Daten |
|---|---|---|---|
| 2 | 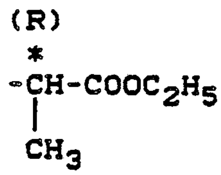 | 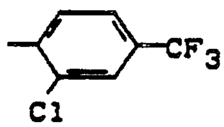 | |
| 3 | 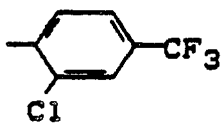 | 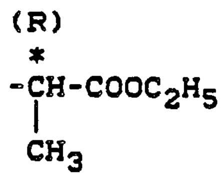 | |
| 4 | 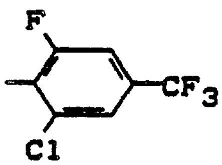 | 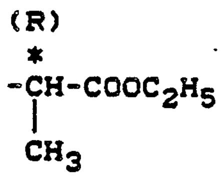 | 7,6 *) |
| 5 | 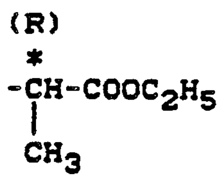 | 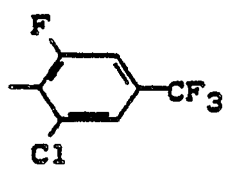 | 7,7 *) |

Tabelle 1 - Fortsetzung

| Beisp.-Nr. | $A^2$ | $A^1$ | Physikalische Daten |
|---|---|---|---|
| 6 | 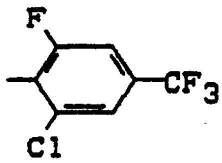 | $-CH_2COOC_2H_5$ | |
| 7 | $-CH_2COOCH_3$ | 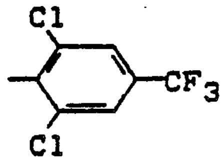 | Fp: 103° C |
| 8 | $-CH_2COOCH_3$ | 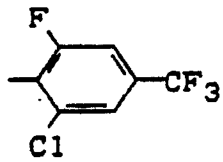 | 4,75 **) |
| 9 | 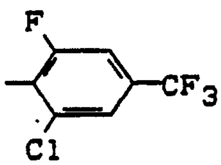 | $-CH_2COOCH_3$ | Fp: 115° C |

*) $^1$H-NMR δ-Werte (ppm) für 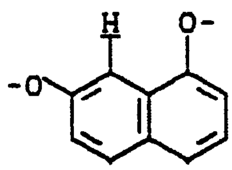

**) $^1$H-NMR δ-Wert (ppm) für $-O-CH_2-COOCH_3$

Ausgangsstoffe der Formel (II)

Beispiel (II-1)

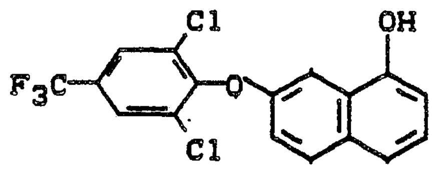

23,4 g (0,095 Mol) 3,4,5-Trichlor-benzotrifluorid werden zu einer auf 120 °C erwärmten Mischung aus 28,4 g (0,18 Mol) 1,7-Dihydroxy-naphthalin, 10,1 g (0,18 Mol) Kaliumhydroxid-Pulver und 100 ml Dimethylsulfoxid langsam unter Rühren gegeben und das Reaktionsgemisch wird dann noch 10 Stunden bei 120 °C gerührt. Nach dem Abkühlen auf 20 °C wird mit 1N-Salzsäure angesäuert, mit Toluol ausgeschüttelt, die organische Phase abgetrennt, mit Wasser gewaschen, mit Natriumsulfat getrocknet und filtriert. Vom Filtrat wird das Lösungsmittel im Wasserstrahlvakuum sorgfältig abdestilliert.

Man erhält 14,9 g (42 % der Theorie) 7-(2,6-Dichlor-4-trifluormethyl-phenoxy)-1-naphthol als öligen Rückstand. $^{1}$H-NMR: $\delta$ = 7,7 ppm für

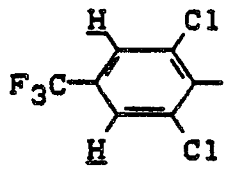

In analoger Weise können auch die übrigen Ausgangsstoffe der Formel (II) hergestellt werden. Falls hierbei zunächst Gemische der beiden möglichen stellungsisomeren Naphtholderivate anfallen, so können diese nach üblichen Methoden, z.B. durch Chromatographie oder durch fraktioniertes Umkristallisieren, getrennt werden.

## Anwendungsbeispiele

In den folgenden Anwendungsbeispielen wird die nachstehend aufgeführte Verbindung als Vergleichssubstanz herangezogen:

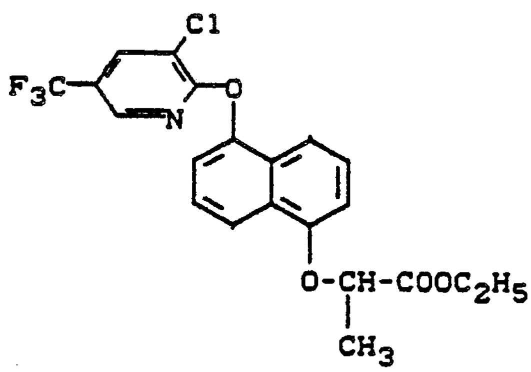

(A)

α-(5-(3-Chlor-5-trifluormethyl-pyridin-2-yl-oxy)-naphthalin-1-yl-oxy)-propionsäure-ethylester (bekannt aus JP-A 79-32477).

### Beispiel A

Post-emergence-Test

| | | |
|---|---|---|
| Lösungsmittel: | 5 | Gewichtsteile Aceton |
| Emulgator: | 1 | Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

| | |
|---|---|
| 0 % = | keine Wirkung (wie unbehandelte Kontrolle) |
| 100 % = | totale Vernichtung |

Eine deutliche Überlegenheit in der Wirksamkeit, insbesondere bei der Bekämpfung von dikotylen Unkräutern, gegenüber der Vergleichssubstanz (A) zeigen in diesem Test z.B. die Verbindungen gemäß Herstellungsbeispiel (1), (5), (7) und (8).

## Ansprüche

1. 1,7-Dioxy-naphthalin-Derivate der allgemeinen Formel (I)

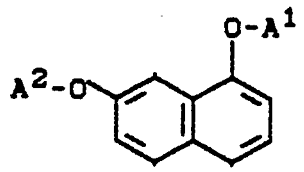

(I)

in welcher
$A^1$ und $A^2$ in jedem Einzelfall verschieden sind und jeweils der eine der beiden Reste ($A^1$ oder $A^2$) für die Gruppierung

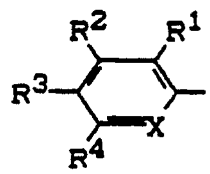

steht, worin
$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy, Trifluormethylthio oder Trifluormethylsulfonyl steht,
$R^4$ für Wasserstoff oder Halogen steht, und
X für Stickstoff oder die Gruppierung C-$R^5$ steht, worin
$R^5$ für Wasserstoff oder Halogen steht,
und jeweils der andere der beiden Reste ($A^2$ oder $A^1$) für die Gruppierung -$A^3$-Z steht, worin
$A^3$ für gegebenenfalls verzweigtes Alkandiyl steht und
Z für Cyano oder die Gruppierung -CO-Y steht, worin
Y für Halogen, Hydroxy, Amino, Alkylamino, Alkenylamino, Alkinylamino, Arylamino, Aralkylamino, Alkoxycarbonylalkylamino, Cyanamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung -O-$R^6$ steht, worin
$R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Arylthioalkyl, Arylalkoxyalkyl, Arylalkylthioalkyl, Trialkylsilylalkyl, Alkoxycarbonylalkyl, Alkylaminocarbonylalkyl, Arylaminocarbonylalkyl, N-Alkyl-N-aryl-aminocarbonylalkyl, Aralkyl, Azolylalkyl, Alkylidenamino oder für ein Ammonium-, Alkylammonium-, Alkalimetall- oder Erdalkalimetall-äquivalent
steht oder für die Gruppierung

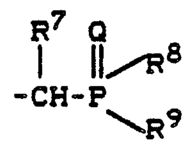

steht, worin
$R^7$ für Wasserstoff, Alkyl, Aryl, Furyl, Thienyl oder Pyridyl steht,
$R^8$ für Alkyl oder Alkoxy steht,
$R^9$ für Alkoxy steht und
Q für Sauerstoff oder Schwefel steht,
oder
$R^6$ weiterhin für die Gruppierung $-(CH_2)_n-R^{10}$ steht, worin
$R^{10}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und
n für die Zahlen 0, 1 oder 2 steht.

2. 1,7-Dioxy-naphthalin-Derivate der allgemeinen Formel (I) gemäß Anspruch 1, dadurch gekennzeichnet, daß darin
$A^1$ und $A^2$ in jedem Einzelfall verschieden sind und jeweils einer der beiden Reste ($A^1$ oder $A^2$) für die Gruppierung

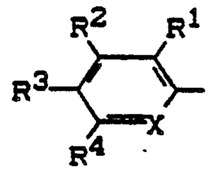

steht, worin
$R^1$ für Wasserstoff, Fluor, Chlor, Brom, Cyano oder Trifluormethyl steht,
$R^2$ für Wasserstoff, Fluor oder Chlor steht,
$R^3$ für Fluor, Chlor, Brom, Trifluormethyl, Trifluormethoxy, Trifluormethylthio oder Trifluormethylsulfonyl steht,
$R^4$ für Wasserstoff, Fluor oder Chlor steht,
X für Stickstoff oder die Gruppierung C-$R^5$ steht, worin
$R^5$ für Wasserstoff, Fluor, Chlor oder Brom steht,
und jeweils der andere der beiden Reste ($A^2$ oder $A^1$) für die Gruppierung -$A^3$-Z steht, worin
$A^3$ für gegebenenfalls verzweigtes $C_1$-$C_4$-Alkandiyl steht und
Z für Cyano oder die Gruppierung -CO-Y steht, worin
Y für Chlor, Hydroxy, Amino, $C_1$-$C_6$-Alkylamino, $C_3$-$C_4$-Alkenylamino, $C_3$-$C_4$-Alkinylamino, Phenylamino, Benzylamino, $C_1$-$C_4$-Alkoxycarbonyl-$C_1$-$C_2$-alkylamino, Cyanamino, Di-($C_1$-$C_4$-alkyl)-amino, Di-($C_3$-$C_4$-alkenyl)-amino, $C_1$-$C_4$-Alkylsulfonylamino, Phenylsulfonylamino, Tolylsulfonylamino, Hydroxyamino, $C_1$-$C_6$-Alkoxyamino, Hydrazino, $C_1$-$C_4$-Alkylsulfonylhydrazino, Phenylsulfonylhydrazino, Tolylsulfonylhydrazino, $C_1$-$C_4$-Alkylthio, Phenylthio, Benzylthio, $C_1$-$C_4$-Alkoxy-carbonyl-$C_1$-$C_2$-alkylthio oder für die Gruppierung -O-$R^6$ steht, worin
$R^6$ für einen gegebenenfalls durch Fluor und/ oder Chlor substituierten Rest aus der Reihe $C_1$-$C_6$-Alkyl, $C_3$-$C_4$-Alkenyl, $C_3$-$C_4$-Alkinyl, $C_1$-$C_4$-Alkoxy-$C_1$-$C_4$-alkyl, $C_1$-$C_4$-Alkylthio-$C_1$-$C_4$-alkyl, $C_1$-$C_4$-Alkylsulfinyl-$C_1$-$C_4$-alkyl, $C_1$-$C_4$-Alkylsulfonyl-$C_1$-$C_4$-alkyl, Phenoxy-$C_1$-$C_3$-alkyl, Trimethylsilylmethyl, Phenylthio-$C_1$-$C_3$-alkyl,

Benzyloxy-$C_1$-$C_3$-alkyl, Benzylthio-$C_1$-$C_3$-alkyl, $C_1$-$C_4$-Alkoxycarbonyl-$C_1$-$C_2$-alkyl, $C_1$-$C_4$-Alkylaminocarbonyl-$C_1$-$C_2$-alkyl, Phenylaminocarbonyl-$C_1$-$C_4$-alkyl, N-($C_1$-$C_4$-Alkyl)-N-phenyl-aminocarbonyl-$C_1$-$C_4$-alkyl, Benzyl, Pyrazolyl-$C_1$-$C_4$-alkyl, $C_2$-$C_4$-Alkylidenamino oder für ein Ammonium-, ein $C_1$-$C_4$-Alkylammonium-, ein Natrium-, Kalium- ode Calcium-äquivalent steht,
oder für die Gruppierung

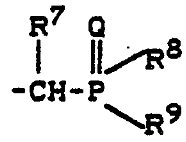

steht, worin
$R^7$ für Wasserstoff, $C_1$-$C_4$-Alkyl, Phenyl, Furyl, Thienyl oder Pyridyl steht,
$R^8$ für $C_1$-$C_4$-Alkyl oder $C_1$-$C_4$-Alkoxy steht,
$R^9$ für $C_1$-$C_4$-Alkoxy steht und
Q für Sauerstoff oder Schwefel steht,
oder
$R^6$ weiterhin für die Gruppierung $-(CH_2)_n-R^{10}$ steht, worin
n für die Zahlen 0, 1 oder 2 steht und
$R^{10}$ für einen gegebenenfalls durch Fluor, Chlor, Brom und/oder $C_1$-$C_4$-Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht.

3. 1,7-Dioxy-naphthalin-Derivate der allgemeinen Formel (I) gemäß Anspruch 1, dadurch gekennzeichnet, daß darin
$A^1$ und $A^2$ in jedem Einzelfall verschieden sind und jeweils einer der beiden Reste ($A^1$ oder $A^2$) für die Gruppierung

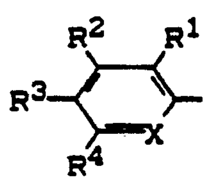

steht, worin
$R^1$ für Cyano, Fluor oder Chlor steht,
$R^2$ für Wasserstoff, Fluor oder Chlor steht,
$R^3$ für Chlor, Trifluormethyl oder Trifluormethylsulfonyl steht,
$R^4$ für Wasserstoff, Fluor oder Chlor steht,
X für Stickstoff oder die Gruppierung C-$R^5$ steht, worin
$R^5$ für Wasserstoff, Fluor oder Chlor steht,
und jeweils der andere der beiden Reste ($A^2$ oder $A^1$) für die Gruppierung -$A^3$-Z steht, worin
$A^3$ für Methylen ($-CH_2-$), Dimethylen ($-CH_2CH_2-$), Trimethylen $(-CH_2-)_3$ oder
Ethyliden

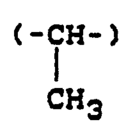

steht und
Z für Cyano oder die Gruppierung -CO-Y steht, worin
Y für Chlor, Hydroxy, Amino, $C_1$-$C_4$-Alkylamino, Phenylamino, $C_1$-$C_4$-Alkoxy-carbonyl-$C_1$-$C_2$-alkylamino, Di-($C_1$-$C_3$-alkyl)-amino, Diallylamino, $C_1$-$C_4$-Alkylsulfonylamino, Phenylsulfonylamino, Hydroxyamino, Cyanamino, $C_1$-$C_4$-Alkoxyamino, Hydrazino, $C_1$-$C_4$-Alkylsulfonylhydrazino, Phenylsulfonylhydrazino, $C_1$-$C_4$-Alkylthio oder $C_1$-$C_4$-Alkoxy-carbonyl-$C_1$-$C_2$-alkylthio oder für die Gruppierung -O-$R^6$ steht, worin

$R^6$ für $C_1$-$C_4$-Alkyl, $C_1$-$C_2$-Alkoxy-$C_1$-$C_2$-alkyl, $C_1$-$C_2$-Alkylthio-$C_1$-$C_2$-alkyl, $C_1$-$C_2$-Alkylsulfinyl-$C_1$-$C_2$-alkyl, $C_1$-$C_2$-Alkylsulfonyl-$C_1$-$C_2$-alkyl, Benzyloxy-$C_1$-$C_3$-alkyl, Benzylthio-$C_1$-$C_3$-Alkyl, $C_1$-$C_4$-Alk oxy-carbonyl-$C_1$-$C_2$-alkyl, $C_1$-$C_4$-Alkylamino-carbonyl-$C_1$-$C_2$-alkyl, Benzyl, Trimethylsilylmethyl oder für ein Ammonium-, $C_1$-$C_3$-Alkylammonium-, Natrium-, oder Kalium-äquivalent steht, oder für die Gruppierung

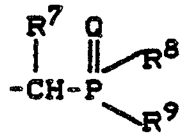

steht, worin
$R^7$ für Wasserstoff, Methyl, Phenyl, Furyl, Thienyl oder Pyridyl steht,
$R^8$ für Methoxy oder Ethoxy steht,
$R^9$ für Methoxy oder Ethoxy steht und
Q für Sauerstoff oder Schwefel steht
oder
$R^6$ weiterhin für die Gruppierung $(-CH_2-)_nR^{10}$ steht, worin
n für die Zahlen 0, 1 oder 2 steht und
$R^{10}$ für einen gegebenenfalls durch Chlor und/oder Methyl substituierten heterocyclischen Rest aus der Reihe Furyl, Tetrahydrofuryl, Thienyl, Perhydropyranyl, Oxazolyl, Thiazolyl und Dioxolanyl steht.

4. Verfahren zur Herstellung von 1,7-Dioxy-naphthalin-Derivaten der Formel (I) gemäß Anspruch 1, dadurch gekennzeichnet, daß man
(a) 7-substituierte 1-Naphthole der allgemeinen Formel (II)

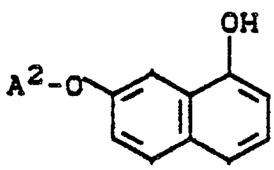

(II)

in welcher
$A^2$ die oben angegebene Bedeutung hat,
mit Verbindungen der allgemeinen Formel (III)
$Z^1 - A^1$ (III)
in welcher
$A^1$ die oben angegebene Bedeutung hat und
$Z^1$ für eine nucleophile Abgangsgruppe steht,
gegebenenfalls in Gegenwart eines Säureakzeptors und gegebenenfalls in Gegenwart eines Verdünnungsmittels umsetzt, und/oder daß man
(b) 8-substituierte 2-Naphthole der allgemeinen Formel (IV)

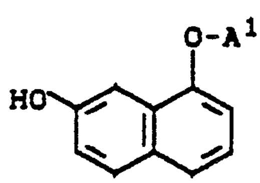

(IV)

in welcher
$A^1$ die oben angegebene Bedeutung hat,
mit Verbindungen der allgemeinen Formel (V)
$A^2 - Z^2$ (V)
in welcher
$A^2$ die oben angegebene Bedeutung hat und
$Z^2$ für eine nucleophile Abgangsgruppe steht gegebenenfalls in Gegenwart eines Säureakzeptors und gegebenenfalls in Gegenwart eines Verdünnungsmittels umsetzt

und gegebenenfalls an den so erhaltenen Verbindungen der Formel (I) weitere Derivatisierungen in dem durch die obige Substituentendefinition vorgegebenen Rahmen nach üblichen Methoden durchführt.

5. Herbizide Mittel, gekennzeichnet durch einen Gehalt an mindestens einem 1,7-Dioxy-naphthalin-Derivat der Formel (I) gemäß Anspruch 1.

6. Verfahren zur Bekämpfung von Unkraut, dadurch gekennzeichnet, daß man 1,7-Dioxy-naphthalin-Derivate der Formel (I) gemäß Anspruch 1 auf das Unkraut und/oder seinen Lebensraum einwirken läßt.

7. Verwendung von 1,7-Dioxy-naphthalin-Derivaten der Formel (I) gemäß Anspruch 1 zur Bekämpfung von Unkraut.

8. Verfahren zur Herstellung von herbiziden Mitteln, dadurch gekennzeichnet, daß man 1,7-Dioxy-naphthalin-Derivate der Formel (I) gemäß Anspruch 1 mit Streckmitteln und/oder oberflächenaktiven Mitteln vermischt.

9. 7-substituierte 1-Naphthole der allgemeinen Formel (II)

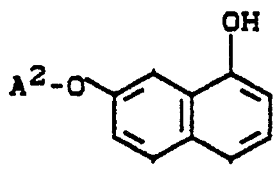

(II)

in welcher
$A^2$ für die Gruppierung

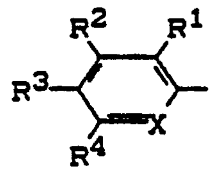

steht, worin
$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy, Trifluormethylthio oder Trifluormethylsulfonyl steht,
$R^4$ für Wasserstoff oder Halogen steht, und
X für Stickstoff oder die Gruppierung C-$R^5$ steht, worin
$R^5$ für Wasserstoff oder Halogen steht,
oder für die Gruppierung -$A^3$-Z steht, worin
$A^3$ für gegebenenfalls verzweigtes Alkandiyl steht und
Z für Cyano oder die Gruppierung -CO-Y steht, worin
Y für Halogen, Hydroxy, Amino, Alkylamino, Alkenylamino, Alkinylamino, Arylamino, Aralkylamino, Alkoxycarbonylalkylamino, Cyanamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung -O-$R^6$ steht, worin
$R^6$ für einen gegebenenfalls durch Halogen substituierten Rest aus der Reihe Alkyl, Alkenyl, Alkinyl, Alkoxyalkyl, Alkylthioalkyl, Alkylsulfinylalkyl, Alkylsulfonylalkyl, Aryloxyalkyl, Arylthioalkyl, Arylalkoxyalkyl, Arylalkylthioalkyl, Trialkylsilylalkyl, Alkoxycarbonylalkyl, Alkylaminocarbonylalkyl, Arylaminocarbonylalkyl, N-Alkyl-N-aryl-aminocarbonylalkyl, Aralkyl, Azolylalkyl, Alkylidenamino oder für ein Ammonium-, Alkylammonium-, Alkalimetall-oder Erdalkalimetall-äquivalent steht oder für die Gruppierung

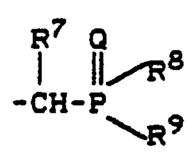

steht, worin
$R^7$ für Wasserstoff, Alkyl, Aryl, Furyl, Thienyl oder Pyridyl steht,

$R^8$ für Alkyl oder Alkoxy steht,
$R^9$ für Alkoxy steht und
Q für Sauerstoff oder Schwefel steht,
oder
$R^6$ weiterhin für die Gruppierung $-(CH_2)_n-R^{10}$ steht, worin
$R^{10}$ für einen gegebenenfalls durch Halogen und/oder Alkyl substituierten heterocyclischen Rest aus der Reihe Furyl, Tetrahydrofuryl, Oxotetrahydrofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und
n für die Zahlen 0, 1 oder 2 steht.

10. 8-substituierte 2-Naphthole der allgemeinen Formel (IV)

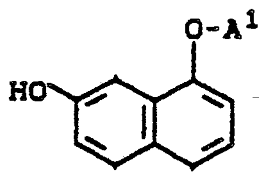

(IV)

in welcher
$A^1$ für die Gruppierung

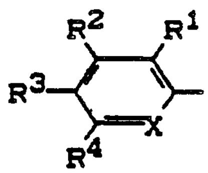

steht, worin
$R^1$ für Wasserstoff, Halogen, Cyano oder Trifluormethyl steht,
$R^2$ für Wasserstoff oder Halogen steht,
$R^3$ für Halogen, Trifluormethyl, Trifluormethoxy, Trifluormethylthio oder Trifluormethylsulfonyl steht,
$R^4$ für Wasserstoff oder Halogen steht, und
X für Stickstoff oder die Gruppierung $C-R^5$ steht, worin
$R^5$ für Wasserstoff oder Halogen steht,
oder für die Gruppierung $-A^3-Z$ steht, worin
$A^3$ für gegebenenfalls verzweigtes Alkandiyl steht und
Z für Cyano oder die Gruppierung -CO-Y steht, worin
Y für Halogen, Hydroxy, Amino, Alkylamino, Alkenylamino, Alkinylamino, Arylamino, Aralkylamino, Alkoxycarbonylalkylamino, Cyanamino, Dialkylamino, Dialkenylamino, Alkylsulfonylamino, Arylsulfonylamino, Hydroxyamino, Alkoxyamino, Hydrazino, Alkylsulfonylhydrazino, Arylsulfonylhydrazino, Alkylthio, Arylthio, Aralkylthio, Alkoxycarbonylalkylthio oder für die Gruppierung $-O-R^6$ steht, worin
$R^6$ für einen gegebenenfalls durch Halogen substituierten Rest aus der Reihe Alkyl, Alkenyl, Alkinyl, Alkoxyalkyl, Alkylthioalkyl, Alkylsulfonylalkyl, Alkylsulfonylalkyl, Aryloxyalkyl, Arylthioalkyl, Arylalkoxyalkyl, Arylalkylthioalkyl, Trialkylsilylalkyl, Alkoxycarbonylalkyl, Alkylaminocarbonylalkyl, Arylaminocarbonylalkyl, N-Alkyl-N-aryl-aminocarbonylalkyl, Aralkyl, Azolylalkyl, Alkylidenamino oder für ein Ammonium-, Alkylammonium-, Alkalimetall-oder Erdalkalimetall-äquivalent
steht oder für die Gruppierung

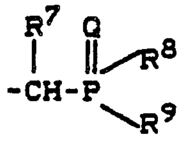

steht, worin
$R^7$ für Wasserstoff, Alkyl, Aryl, Furyl, Thienyl oder Pyridyl steht,

$R^8$ für Alkyl oder Alkoxy steht,
$R^9$ für Alkoxy steht und
Q für Sauerstoff oder Schwefel steht,
oder
$R^6$ weiterhin für die Gruppierung $-(CH_2)_n-R^{10}$ steht, worin
$R^{10}$ für einen gegebenenfalls durch Halogen und/oder Alkyl substituierten heterocyclischen Rest aus der Reihe Furyl, Tetrahydrofuryl, Oxotetrahy drofuryl, Thienyl, Tetrahydrothienyl, Perhydropyranyl, Oxazolyl, Thiazolyl, Thiadiazolyl, Dioxolanyl, Perhydropyrrolyl, Oxoperhydropyrrolyl, Pyridinyl oder Pyrimidinyl steht und
n für die Zahlen 0, 1 oder 2 steht.

Europäisches Patentamt

# EUROPÄISCHER RECHERCHENBERICHT

Nummer der Anmeldung

EP 89110529.8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE - A1 - 3 525 562 (SCHERING) * Ansprüche * -- | 1,4-10 | C 07 C 69/712<br>C 07 C 43/275<br>C 07 D 213/64<br>A 01 N 39/02<br>A 01 N 43/40 |
| D,A | CHEMICAL ABSTRACTS, Band 91, Nr. 11, 10. September 1979, Columbus, Ohio, USA R.NISHIYAMA et al. "Pyridyloxynaphthoxypropionic acid derivatives" Seite 767, Spalte 2, Zusammenfassung-Nr. 91 510j & Jpn. Kokai Tokkyo Koho 79,32477 ---- | 1,4,5 | |

| RECHERCHIERTE SACHGEBIETE (Int. Cl.4) |
|---|
| C 07 C 69/00<br>C 07 C 43/00<br>C 07 D 213/00 |

Der vorliegende Recherchenbericht wurde für alle Patentansprüche erstellt.

| Recherchenort | Abschlußdatum der Recherche | Prüfer |
|---|---|---|
| WIEN | 15-09-1989 | HOFBAUER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82